# EUROPÄISCHE PATENTSCHRIFT

(11) **EP 1 115 726 B1**
(45) Veröffentlichungstag und Bekanntmachung des Hinweises auf die Patenterteilung: **27.11.2002**
(21) Anmeldenummer: 99942890.7
(22) Anmeldetag: 20.08.1999
(51) Int. Cl.: C07D 487/22

(54) **VERFAHREN ZUR HERSTELLUNG VON METALLOPORPHYRIN-METALLKOMPLEX-KONJUGATEN**
METHOD FOR PREPARING METALLOPORPHYRIN-METAL COMPLEX CONJUGATES
PROCEDE POUR LA PRODUCTION DE CONJUGUES METALLOPORPHYRINE-COMPLEXE METALLIQUE

(30) Priorität: 22.09.1998 DE 19845782
(43) Veröffentlichungstag der Anmeldung: 18.07.2001
(73) Patentinhaber: SCHERING AKTIENGESELLSCHAFT, 13342 Berlin (DE)
(72) Erfinder: PLATZEK, Johannes, D-12621 Berlin (DE); NIEDBALLA, Ulrich, D-14195 Berlin (DE)
(86) Internationale Anmeldenummer: EP9906075
(87) Internationale Veröffentlichungsnummer: WO00017205

(56) Entgegenhaltungen:
- EP-A- 0 336 879
- EP-A- 0 355 041
- DE-A- 4 232 925

## Beschreibung

Die Erfindung betrifft den in den Patentansprüchen gekennzeichneten Gegenstand, das heißt ein Verfahren zur Herstellung von Metalloporphyrin-Metallkomplex-Konjugaten enthaltend mindestens ein Ion eines Elements der Ordnungszahl 20-32, 37-39, 42-51 oder 57-83 im Komplexteil des Konjugats.
Bisher wurden Metalloporphyrin-Metallkomplex-Konjugate nach mehrstufigen Verfahren hergestellt. In EP 0 336 879 werden Tetraphenylporphyrine zuerst mit einem Komplexbildner, dann mit einem Metalloxid oder Metallsalz zur Komplexierung des Komplexbildners und anschließend mit Manganacetat zur Substitution der pyrrolischen NH-Gruppen umgesetzt. Die Umsetzung mit Manganacetat erfolgt unter drastischen Bedingungen bei 80°C in Eisessig. Die Verwendung des Acetates hat den Nachteil, daß weder ein Arbeiten mit Überschüssen noch ein Arbeiten unter wasserfreien Bedingungen möglich ist. Daher ist die Anwendbarkeit dieses Verfahrens begrenzt.
Aus EP 0 355 041 ist ein Verfahren zur Herstellung von Metalloporphyrin-Metallkomplex-Konjugaten bekannt, in dem zuerst das Mangan in den Porphyrinkem eingeführt wird, und anschließend die Umsetzung mit einem Komplexbildner und einem Metalloxid oder Metallsalz zur Komplexierung des Komplexbildners erfolgt. Diese Verfahrensweise hat einen entscheidenden Nachteil. Die Festlegung auf das Metall im Porphyrin erfolgt bereits in der ersten Stufe, so daß für eine Variation des Porphyrinmetalls bei gleichbleibendem Komplexbildner die Synthese für jedes neue Porphyrinmetall jedesmal über zumindest drei Stufen durchgeführt werden muß. Es sind dabei Nachteile wie z.B. Ausbeuteverluste und Auswirkungen im Bereich des Umweltschutzes wie z.B. Erhöhung der Menge der metallhaltigen Abfälle, nicht zu umgehen.

Daher war es die Aufgabe der vorliegenden Erfindung, einen Syntheseweg für Metalloporphyrin-Metallkomplex-Konjugate bereitzustellen, der die oben genannten Nachteile nicht aufweist, vor allem aber eine wirtschaftlichere Syntheseführung bei Variation des Porphyrinmetalls und die Anwesenheit von empfindlichen Gruppen ermöglicht.

Diese Aufgabe wird durch das erfindungsgemäße Verfahren gelöst, wie es in den Patentansprüchen gekennzeichnet ist.
Es handelt sich um ein Verfahren zur Herstellung von Metalloporphyrin-Metallkomplex-Konjugaten, das dadurch gekennzeichnet ist, daß ein Porphyrin-Metallkomplex-Konjugat durch Umsetzung mit einem Metallacetylacetonat in einem protischen oder aprotischen polaren Lösungsmittel oder Lösungsmittelgemisch bei Temperaturen von Raumtemperatur bis 150°C in Konzentrationen von 3-30% in ein Metalloporphyrin-Metallkomplex-Konjugat überführt wird.

Die Einführung des zentralen Metallatoms in den Porphyrinkern erfolgt durch eine Umkomplexierungsreaktion mit Metallacetylacetonat, das heißt, das bereits im Acetylacetonat komplex gebundene Metall wechselt von seinem Komplexliganden Acetylaceton zum Komplexliganden Porphyrin und wird von diesem ebenfalls komplex gebunden.

Eine besondere Ausführungsform der Erfindung ist ein Verfahren zur Herstellung von Metalloporphyrin-Metallkomplex-Konjugaten der allgemeinen Formel I 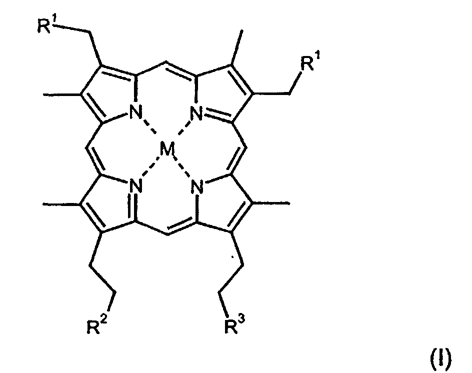 enthaltend mindestens ein Ion eines Elements der Ordnungszahl 20-32, 37-39, 42-51 oder 57-83 im Komplexteil des Moleküls,
worin
- M: für ein Ion der Metalle Magnesium, Aluminium, Chrom, Mangan, Eisen, Cobalt, Nickel, Kupfer, Zink, Gallium, Technetium, Indium, Zinn, Europium, Lutetium, Tallium, Wismut oder für Oxovanadium(IV),
- R¹: für ein Wasserstoffatom, für einen geradkettigen C₁-C₆-Alkylrest, einen C₇-C₁₂-Aralkylrest oder für eine Gruppe OR' steht,
wobei
R' ein Wasserstoffatom oder ein C₁-C₃-Alkylrest ist,
- R²: für R³, eine Gruppe -CO-Z oder eine Gruppe (NH)ₒ-(A)_{q}-NH-D steht,
wobei
Z eine Gruppe -OL ist, mit L in der Bedeutung eines anorganischen oder organischen Kations oder eines C₁-C₄ Alkylrestes,
A eine Phenylenoxy- oder eine durch ein oder mehrere Sauerstoffatome unterbrochene C₁-C₁₂-Alkylen- oder C₇-C₁₂-Aralkylengruppe bedeutet,
o und q unabhängig voneinander die Ziffern 0 oder 1 bedeuten,
D ein Wasserstoffatom oder eine Gruppe -CO-A-(COOL)ₒ-(H)ₘ bedeutet, mit m gleich 0 oder 1 und unter der Maßgabe, daß die Summe aus m und o gleich 1 ist,
- R³: für eine Gruppe -(C=Q)(NR⁴)ₒ-(A)_{q}-(NR⁵)-K steht,
wobei
Q für ein Sauerstoffatom oder für zwei Wasserstoffatome steht,
R⁴ eine Gruppe -(A)_{q}-H bedeutet und
K einen Komplexbildner der allgemeinen Formel (lla), (IIb), (llc), (IId) oder (IIe) bedeutet, und
R⁵ für den Fall, daß K ein Komplexbildner der Formel (IIa) ist, die gleiche Bedeutung wie R⁴ hat und für den Fall, daß K ein Komplexbildner der Formel (IIb), (llc), (IId) oder (lle) ist, die gleiche Bedeutung wie D hat, mit der Maßgabe, daß eine direkte Sauerstoff-Stickstoff Bindung nicht zugelassen ist
und K für einen Komplexbildner der allgemeinen Formeln (lla), (IIb), (llc), (IId) oder (IIe) 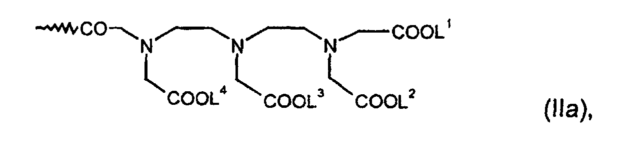 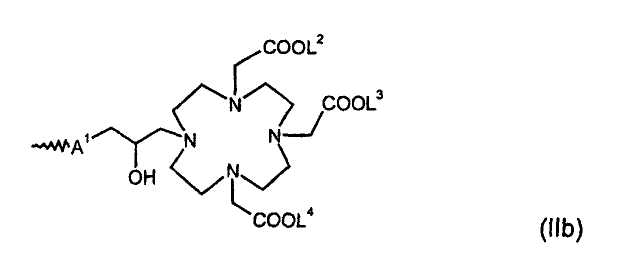 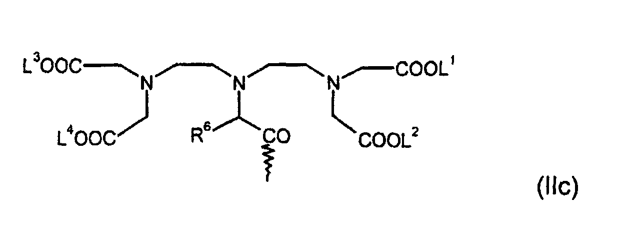 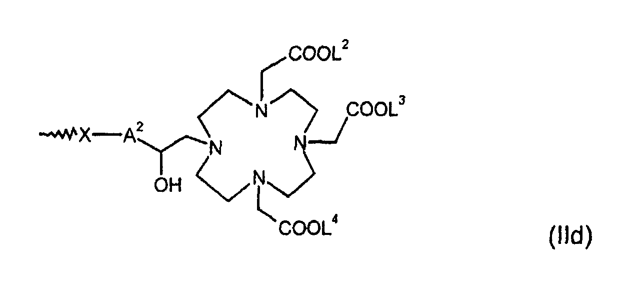 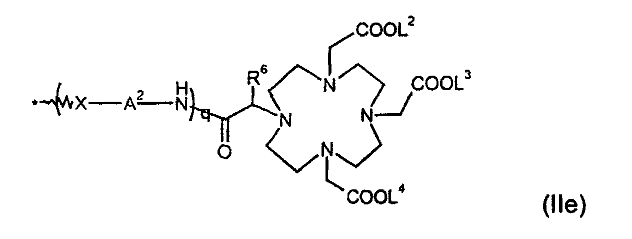 steht,
worin
o und q die oben angegebene Bedeutung haben,
A¹ die für A angegebene Bedeutung hat,
R⁶ für ein Wasserstoffatom, eine geradkettige oder verzweigte C₁-C₇-Alkylgruppe, eine Phenyl- oder Benzylgruppe,
A² für eine Phenylen-,
eine -CH₂-NHCO-CH₂-CH(CH₂COOH)-C₆H₄-β,
eine Phenylenoxy-, oder eine gegebenenfalls durch ein oder mehrere Sauerstoffatome, 1 bis 3 -NHCO- und/oder 1-3-CONH-Gruppen unterbrochene und/oder mit 1 bis 3 -(CH₂)ₒ₋₅COOH-Gruppen substituierte C₁-C₁₂-Alkylen- oder C₇-C₁₂-Aralkylengruppe,
wobei β für die Bindungsstelle an X steht,
X für eine -CO- oder NHCS-Gruppe und
L¹, L², L³ und L⁴ unabhängig voneinander für ein Metallionenäquivalent eines Elements der oben genannten Ordnungszahl steht, unter den Maßgaben, daß mindestens zwei dieser Substituenten für ein Metallionenäquivalent stehen, und daß zum Ausgleich gegebenenfalls vorhandener Ladungen im Metalloporphyrin-Metallkomplex-Konjugat weitere Anionen vorhanden sind und worin freie, nicht zur Komplexbildung benötigte Carbonsäuregruppen auch als Salze mit physiologisch verträglichen anorganischen und/oder organischen Kationen oder als Ester oder als Amide vorliegen können,
das dadurch gekennzeichnet ist, daß
Porphyrin-Metallkomplex-Konjugate der allgemeinen Formel (III) 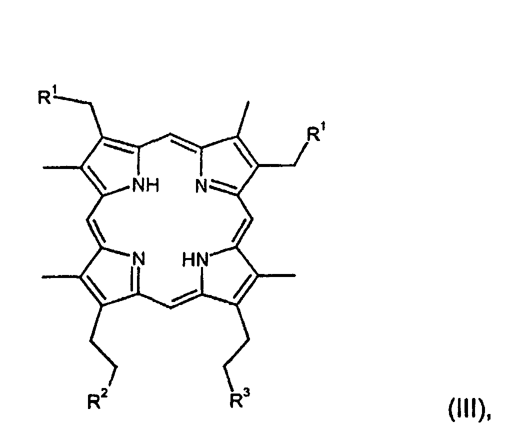 worin R¹, R², R³ und R⁴ die oben angegebenen Bedeutungen haben,
durch Umsetzung mit einem Metallacetylacetonat des Metalls M in einem protischen oder aprotischen polaren Lösungsmittel oder Lösungsmittelgemisch bei Temperaturen von Raumtemperatur bis 150°C in Konzentrationen von 3-30% in Metalloporphyrin-Metallkomplex-Konjugate der Formel (I) überführt werden.

Als Metall M kommen die Metalle Magnesium, Aluminium, Chrom, Mangan, Eisen, Cobalt, Nickel, Kupfer, Zink, Gallium, Technetium, Indium, Zinn, Europium, Lutetium, Tallium, Wismut und Oxovanadium(IV) in Frage. Bevorzugt werden Magnesium, Aluminium, Chrom, Mangan, Eisen, Cobalt, Nickel, Kupfer, Zink, Zinn, Lutetium, und Oxovanadium(IV). Besonders bevorzugt sind Mangan, Eisen, Kupfer und Zink.

Die Metalle liegen im Porphyrin, wenn nicht anders angegeben, als zwei- oder dreiwertige Ionen vor. Sofern eines der im Porphyrin gebundenen Ionen in einer höheren Oxidationsstufe als +2 vorliegt, so wird (werden) die überschüssige(n) Ladung(en) z.B. durch Anionen von organischen oder anorganischen Säuren, bevorzugt durch Acetat-, Tartrat-, Succinat-, Maleat-, Chlorid-, Sulfat- und Nitratlonen oder durch in R² und/oder R³ vorhandene negative Ladungen ausgeglichen.

Bevorzugte Alkylreste R¹ sind geradkettige C₁-C₃ Alkylreste. Besonders bevorzugt ist der Methylrest.

Der Alkylrest R' kann ein Methyl, Ethyl, Propyl oder Isopropylrest sein.

Die Aralkylreste R¹, können Benzyl, oder 4-Methoxybenzyl sein.

Die anorganischen Kationen L sind beispielsweise das Lithium- und das Kalium-Ion, insbesondere das Natrium-Ion.

Die organischen Kationen L sind beispielsweise Kationen organischer Basen; genannt seien Kationen von primären, sekundären oder tertiären Aminen, wie zum Beispiel Ethanolamin, Diethanolamin, Morpholin, Glucamin, N,N-Dimethylglucamin, insbesondere Meglumin.

Der Alkylrest Z kann geradkettig oder verzweigt sein. Methyl und Ethyl sind bevorzugt.

R² und R³ stehen bevorzugt für den gleichen Rest. R² und R³ stehen bevorzugt für die Gruppen -CONHNH-K, -CONH-(CH₂)₂-NH-K, -CONH-(CH₂)₃-NH-K, -CONH-(CH₂)₄-NH-K und -CONH-(CH₂)₂-O-(CH₂)₂-NH-K. Besonders bevorzugt ist die Gruppe -CONHNH-K.

Die Bedeutung eines Sauerstoffatoms wird für Q bevorzugt.

R⁴ ist bevorzugt Wasserstoff.

Bevorzugte Komplexbildner K sind Komplexbildner der Formeln IIa und IIe.

R⁶ steht bevorzugt für ein Wasserstoffatom oder eine Methylgruppe.

A² steht bevorzugt für eine -CH₂-, -(CH₂)₂-, -CH₂OC₆H₅-β, -CH₂OCH₂-, -C₆H₅-, -CH₂-NHCO-CH₂-CH(CH₂COOH)-C₆H₅-β, Gruppe, wobei β für die Bindungestelle an X steht.

X steht bevorzugt für die CO-Gruppe.

Besonders bevorzugte Verbindungen sind:
{µ-[{16,16'-{Zink(II)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}-digadolinato(2-), Dinatrium,
{µ-[{16,16'-[Acetatomangan(III)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}digadolinato(2-), Dinatrium,
{µ-[{16,16'-[Acetatoeisen(III)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}digadolinato(2-), Dinatrium und
{µ-[{16,16'-[Kupfer(II)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}digadolinato(2-), Dinatrium.

Das vorliegende Verfahren ist gegenüber dem Stand der Technik besonders vorteilhaft.
Metallacetylacetonate sind Feststoffe, die sich bequem bei Arbeiten mit Überschüssen von der Reaktionsmischung abfiltrieren lassen, die Acetate dagegen nicht.
Metallacetylacetonate lassen sich im Gegensatz zu z.B. Acetaten sehr gut trocknen, was für eine Reaktionsführung unter Wasserausschluß notwendig ist. Gerade auch für einen Einsatz bei Reaktionen im Betriebsmaßstab ist ein gutes Trocknen von großem Vorteil, da sich bei großen Mengen anhaftendes Wasser schnell subsummiert und den Reaktionsverlauf negativ beeinflußt.
Bei Verwendung von Metallacetylacetonaten ist eine einfache Abtrennung des ersten Metall-Liganden möglich, wenn gewünscht auch ionenfrei, da sich Acetylacetonat auch als Acetylaceton zurückgewinnen und in den Reaktionskreislauf wieder zurückführen läßt. Das aufwendige Abtrennen (z.B. von Acetaten) und Entsorgen von anfallenden Salzen oder anderen Feststoffen, wie z.B. Salicylsäure bei der Verwendung von Salicylaten, entfällt.
Das Rückführen von Acetylaceton in den Reaktionskreislauf ermöglicht eine die Umwelt schonende Reaktionsführung.

Da Überschüsse wie oben erwähnt keine Probleme machen, kann die Reaktionskinetik durch Verwendung von Metallacetylacetonat-Überschüssen beeinflußt werden, das heißt, eine Verkürzung der Reaktionszeiten ist durch Einsatz von Acetylacetonat-Überschüssen möglich.
Wegen des Einbaus des zentralen Metallatoms im letzten Schritt können leicht viele Porphyrine gleichen Gerüstes mit verschiedenen zentralen Metallatomen synthetisiert werden, ohne daß für jedes neue Zentralatom die gesamte Synthese wiederholt werden muß (effizientere Reaktionsführung).
Ein Einbau von unterschiedlichen Metallen mit zweifach- oder dreifach positiver Ladung in den Porphyrinkern ist ohne Störung durch den jeweiligen Metallkomplexliganden möglich.
Der Einbau des zentralen Metallatoms erfolgt ohne Metallaustausch. Aufgrund der hohen thermodynamischen Konstanten von DTPA-Amiden ist es erstaunlich und unerwartet, daß es nicht zu einem Metallaustausch kommt, der zu Mischkomplexen führen würde.
Man erhält das Produkt in hohen Ausbeuten.
Eine besonders hohe Reinheit des Produktes wird erreicht.

Die Metalle werden als Acetylacetonate eingesetzt.

Als Lösungsmittel kommen protische oder aprotische polare Lösungsmittel wie z.B. Essigsäure, Ameisensäure, Wasser, Methanol, Ethanol, Dimethylformamid, Formamid, Dimethylsulfoxid, Pyridin oder Mischungen von zwei oder mehreren der genannten Lösungsmittel in Frage. Bevorzugt werden Eisessig, Wasser und Mischungen beider Lösungsmittel.

Die Reaktionszeit beträgt 1-24 Stunden, bevorzugt 3-12 Stunden, besonders bevorzugt 3-8 Stunden.

Die Reaktionstemperatur liegt in einem Bereich von 20°C bis 150°C. Bevorzugt werden 50°C bis 130°C, besonders bevorzugt 70°C bis 120°C.

Bei einer typischen Isolierung wird das Produkt zunächst gefällt (a) oder die Reaktionsmischung eingedampft (b). Bei der Fällung werden Fällungsmittel wie z.B. Aceton, Diethylether, Diisopropylether, oder Tetrahydrofuran zugesetzt, die Suspension filtriert, das Rohprodukt getrocknet.
Das Rohprodukt aus (a) und (b) wird in Wasser gelöst, auf pH 7,2 eingestellt und gefriergetrocknet. Je nach Reinheit wird dann an Kieselgel oder RP-18 chromatographiert und anschließend nach Kristallisation gefriergetrocknet, dialysiert oder eine Ultrafiltration (z.B. 1000D Membran) durchgeführt.

Die nachfolgenden Beispiele dienen der näheren Erläuterung der Erfindungsgegenstandes, ohne ihn auf diese beschränken zu wollen.

Die für die Synthesen der Metalloporphyrin-Metallkomplex-Konjugate verwendeten Ausgangsporphyrine werden im folgenden beschrieben:

### Synthesevorschrift 1

### {µ[{16,16'-[7,12-Diethyl-3,8,13,17-tetramethyl-porphyrin-2,18-diyl]-bis[3,6,9-tris(carboxvmethyl)-11,14-dioxo-3,6,9,12,13-pentaaza-hexadecanoato]}(8-)]}digadolinato(2-),dinatrium

Die Herstellung erfolgt nach Beispiel 1c aus WO 94/07894.

Ausbeute: 1,19 g (70,1 % d.Th.) rotbraunes Pulver

| Elementaranalyse (berechnet auf wasserfreie Substanz): | | | | | |
|---|---|---|---|---|---|
| ber. | C 43,88 | H 4,51 | Gd 18,53 | Na 2,71 | O 18,85 |
| gef. | C 43,71 | H 4,30 | Gd 18,28 | Na 2,80 | |

### Synthesevorschrift 2a

### {7,12-Diethyl-3,8,13,17-tetramethyl-2,18-bis[15,15-dimethyl-3,6,13-trioxo-8-(2-{N,N-bis[(tert.butoxycarbonyl)methyl]amino}-ethyl)-11-[(tert-butoxycarbonyl)-methyl]14-oxa-4,5,8,11-tetraazahexadec-1-yl}-porphyrin

8,31 g (13,45 mmol) 3,9-Bis(tert-butoxycarbonylmethyl)-6-carboxymethyl-3,6,9-triazaundecandisäure-di-tert-butylester, dargestellt nach DE 19 50 78 19, Beispiel 1f, und 2,09 g (15 mmol) 4-Nitrophenol werden in 60 ml Dimethylformamid gelöst und bei 0°C 5,16 g (25 mmol) N,N-Dicyclohexylcarbodiimid zugegeben. Man rührt 3 Stunden bei 0°C, dann über Nacht bei Raumtemperatur. Zu der so hergestellten Aktivesterlösung tropft man 2 g (3,36 mmol) 3,3'-(7,12-Diethyl-3,8,13,17-tetramethylporphyrin-2,18-diyl)-di(propanohydrazid), dargestellt nach H. Fischer, E. Haarer und F. Stadler, Z. Physiol. Chem., 241, 209 (1936), (gelöst in 50 ml Pyridin) zu und rührt über Nacht. Man dampft im Vakuum zur Trokkene ein und chromatogrphiert den Rückstand an Kieselgel (Laufmittel: Dichlormethan/2-Propanol = 20:1).

Ausbeute: 5,24 g (87% d. Th.) eines dunkelbrunen Feststoffes

| Elementaranalyse (berechnet auf wasserfreie Substanz): | | | |
|---|---|---|---|
| ber. | C 62,92 | H 8,31 | N 10,93 |
| gef. | C 62,81 | H 8,45 | N 10,80 |

### Synthesevorschrift 2b)

### {µ[{13,13'-[7,12-Diethyl-3,8,13,17-tetramethyl-porphyrin-2,18-diyl]-bis{3-carboxymethyl-6-(2-{N,N-bis[(carboxy)methyl]amino}ethyl)-8,11-dioxo-3,6,9,10-tetraazatridecanoato]}(8-)]}digadolinato(2-), dinatrium

5 g (2,79 mmol) der Titelverbindung aus Synthesevorschrift 2a werden in 100 ml Trifluoressigsäure gelöst und 8 Stunden bei Raumtemperatur gerührt. Man dampft im Vakuum ur Trockene ein. Der so erhaltene Ligand wird in 100 ml Wasser gelöst und 1,01 g (2,79 mmol) Gadoliniumoxid zugegeben. Man rührt bei 60°C und hält durch Zugabe von 1 N aqu. Natronlauge den pH-Wert bei 5. Die Lösung wird filtriert und das Filtrat mit 1 N aqu. Natronlauge auf pH 7,2 eingestellt. Anschließend wird an RP 18 chromatographiert (Laufmittel: Gradient aus Wasser/Acetonitril).

Ausbeute: 4,40 g (95% d. Th.) eines amorphen Feststoffes
Wassergehalt: 10,3%

| Elementaranalyse (berechnet auf wasserfreie Substanz): | | | | | |
|---|---|---|---|---|---|
| ber. | C 43,86 | H 4,51 | N 11,55 | Gd 18,52 | Na 2,71 |
| gef. | C 43,61 | H 4,70 | N 11,38 | Gd 18,37 | Na 2,50 |

### Synthesevorschrift 3

### {10,10'-(µ-{10,10'-(7,12-diethyl-3,8,13,17-tetramethylporphyrin-2,18-diyl)bis[(1RS)-1-methyl-2,5,8-trioxo-3,6,7-triaza-dec-1-yl]})bis[1,4,7,10-tetraazacyclododecan-1,4,7-triacetato(3-)]}digadolinium

8,47 g (13,45 mmol) des Gd-Komplexes der 10-(4-Carboxy-2-oxo-3-aza-1-methyl-butyl)-1,4,7,10-tetraazacyclododecan-1,4,7-triessigsäure, hergestellt nach PCT/EP 97/06593, 0,64 g Lithiumchlorid (15 mmol) und 2,09 g (15 mmol) 4-Nitrophenol werden in 100 ml Dimethylsulfoxid bei 50°C gelöst. Nach Abkühlen auf Raumtemperatur werden 5,16 g (25 mmol) N,N'-Dicyclohexylcarbodiimid zugesetzt und 12 Stunden voraktiviert. Zu der so hergestellten Lösung gibt man 2 g (3,36 mmol) 3,3'-(7,12-Diethyl-3,8,13,17-tetramethylporphyrin-2,18-diyl)di(propanohydrazid) und 0,71 g (7 mmol) Triethylamin und rührt über Nacht bei Raumtempartur. Die erhaltene Suspension wird anschließend mit ausreichend Aceton bis zur Fällung versetzt, der Niederschlag abgesaugt, getocknet, in Wasser aufgenommen, von unlöslichem Dicyclohexylhamstoff abfiltriert und das Filtrat an RP 18 chromatographiert (Laufmittel: Gradient aus Tetrahydrofuran/Wasser).

Ausbeute: 5,07 g (83% d. Th.) eines dunkelbraunen amorphen Pulvers
Wassergehalt: 7,9%

| Elementaranalyse (berechnet auf wasserfreie Substanz): | | | | |
|---|---|---|---|---|
| ber. | C 47,56 | H 5,43 | N 13,87 | Gd 17,30 |
| gef. | C 47,42 | H 5,53 | N 13,68 | Gd 17,15 |

### Synthesevorschrift 4

### Konjugat aus 3,3'-(7,12-Diethyl-3,8,13,17-tetramethylporphyrin-2-18-diyl)di(propanohydrazid) und dem 10-[7-(4-lsothiocyanatophenyl)-2-hydroxy-5-oxo-7-(carboxymethyl)-4-aza-heptyl)]-1,4,7-tris(carboxylatomethyl)-1,4,7,10-tretraazacyclododecan, Gadoliniumkomplex, Natriumsalz

### ({10,10'-{µ-[(7,12-Diethyl-3,8,13,17-tetramethylporphyrin-2,18-diyl)bis{(1-oxopropan-3,1-diyl)hydrazino-thiocarbonylamino-4,1-phenylen[(3RS)-3-carboxymethyl-1-oxopropan-3,1-diyl]amino(2-hydroxypropan-3,1-diyl)}]}bis[1,4,7,10-tetraazacyclododecan-1,4,7-triacetato(4-)]}digadolinium,dinatrium

Zu 594,8 mg (1 mmol) 3,3'-(7,12-Diethyl-3,8,13,17-tetramethylpophyrin-2,18-diyl)di(propanohydrazid) und 1806 mg (2,2 mmol) 10-[7-(4-lsothiocyanatophenyl)-2-hydroxy-5-oxo-7-(carboxymethyl)-4-aza-heptyl)-1,4,7-tris(carboxylatomethyl)-1,4,7,10-tetraazacyclododecan, Gd-Komplex, Natriumsalz, hergestellt nach WO 94/07894, Beispiel 1, in 50 ml Wasser gibt man 1,01 g (10 mmol) Triethylamin und rührt 12 Stunden bei Raumtemperatur. Man dampft im Vakuum zur Trockene ein und chromatographiert an Kieselgel (Laufmittel: Methanol/Wasser/Eisessig = 10/5/1). Die produkthaltigen Fraktionen werden zur Trockene eingedampft, der Rückstand in 100 ml Wasser gelöst und mit 2 N Natronlauge auf pH 7,2 gestellt. Anschließend wird gefriergetrocknet.

Ausbeute: 2,03 g (89% d.Th.) eines dunkelbraunen Pulvers
Wassergehalt: 7,9 %

| Elementaranalyse (berechnet auf wasserfreie Substanz): | | | | | | |
|---|---|---|---|---|---|---|
| ber. | C 48,45 | H 5,22 | N 12,28 | S 2,81 | Gd 13,79 | Na 2,02 |
| gef. | C 48,37 | H 5,37 | N 12,15 | S 2,72 | Gd 13,58 | Na 1,75 |

### Beispiel 1

### Allgemeine Herstellungsvorschrift zur Herstellung der Metalloporphyrin-Metallkomplex-Konjugaten aus den Porphyrin-Metallkomplex-Konjugaten aus den Vorschriften 1 bis 4 am Beispiel eines Porphyrin-Metallkomplex-Konjugats aus Vorschrift 1

3 g (17,3 mmol) der Verbindung aus Synthesevorschrift 1b) (Porphyrin des Typs A) werden in 250 ml Essigsäure gegeben und mit 0,450 g (17,3 mmol, oder gegebenenfalls bis zu 30% Überschuß) Kupfer(II)acetonylacetonat, versetzt. Es wird 3,5 Stunden auf 119°C erwärmt, wobei Lichtausschluß herrscht und unter Stickstoff gearbeitet wird.
Dann wird im Vakuum eingeengt, Essigsäurereste durch Kodestillation mit Toluol entfernt, mit Essigester digeniert und der Feststoff abgesaugt. Der Feststoff wird in Wasser gelöst und an Kieselgel RP-18 chromatographiert. Eluiert wird mit einem Gradienten aus Wasser/Methanol. Die produkthaltigen Fraktionen werden im Vakuum zur Trockene eingeengt. Der Rückstand wird in Wasser gelöst und mit 1N Natronlauge auf einen pH-Wert von 7,2 eingestellt. Es wird filtriert und das Produkt durch Lyophilisation gewonnen.
Ausbeute: 3,05 g (98% d.Th.)

| Elementaranalyse (berechnet auf wasserfreie Substanz): | | | | | | |
|---|---|---|---|---|---|---|
| ber. | C 42,33 | H 4,24 | N 11,15 | Cu 3,61 | Gd 17,88 | Na 2,61 |
| gef. | C 42,45 | H 4,31 | N 11,00 | Cu 3,70 | Gd 18,01 | Na 2,71 |

Die in den Tabellen 1-4 aufgeführten Verbindungen wurden nach dieser allgemein anwendbaren Herstellungsvorschrift synthetisiert.
Tabelle 1: Einbau von Metallionen als Zentralatom in den Gd-Komplex von Porphyrinen, die nach Vorschrift 1 hergestellt wurden.
Tabelle 2: Einbau von Metallionen als Zentralatom in den Gd-Komplex von Porphyrinen, die nach Vorschrift 2 hergestellt wurden.
Tabelle 3: Einbau von Metallionen als Zentralatom in den Gd-Komplex von Porphyrinen, die nach Vorschrift 3 hergestellt wurden.
Tabelle 4: Einbau von Metallionen als Zentralatom in den Gd-Komplex von Porphyrinen, die nach Vorschrift 4 hergestellt wurden.

### Beispiel 2

### {µ[{16,16'-[Kupfer(II)-7,12-diethyl-3,8,13,17-tetramethylporphyrin-2,18-diyl]-bis[3,6,9-tris(carboxymethyl)-11,14-dioxo-3,6,9,12,13-pentaazahexadecaboato]}(8-)]}-digadolinato(2-), Dinatrium

In 500 I Eisessig werden 25,0 kg (14,72 mol) der Verbindung aus Synthesevorschrift 1b) gelöst, mit 3854,3 g (14,72 mol) Kupfer(II)acetylacetonqat anteilweise unter Rühren versetzt und unter Lichtausschluß und Abdeckung mit Stickstoff 6 Stunden auf 120°C erhitzt. Dann wird im Vakuum eingeengt, Essigsäurereste weitgehend durch Kodestillation mit Toluol entfernt, der Rückstand mit Essigester digeniert, der Feststoff abgesaugt und getrocknet bei 70°C im Umlufttrokkenschrank.
Ausbeute: 25,12 kg (97% d.Th.)

| Elementaranalyse (berechnet auf wasserfreie Substanz): | | | | | | |
|---|---|---|---|---|---|---|
| ber. | C 42,33 | H 4,24 | N 11,15 | Cu 3,61 | Gd 17,88 | Na 2,61 |
| gef. | C 42,46 | H 4,36 | N 11,01 | Cu 3,56 | Gd 17,79 | Na 2,70 |

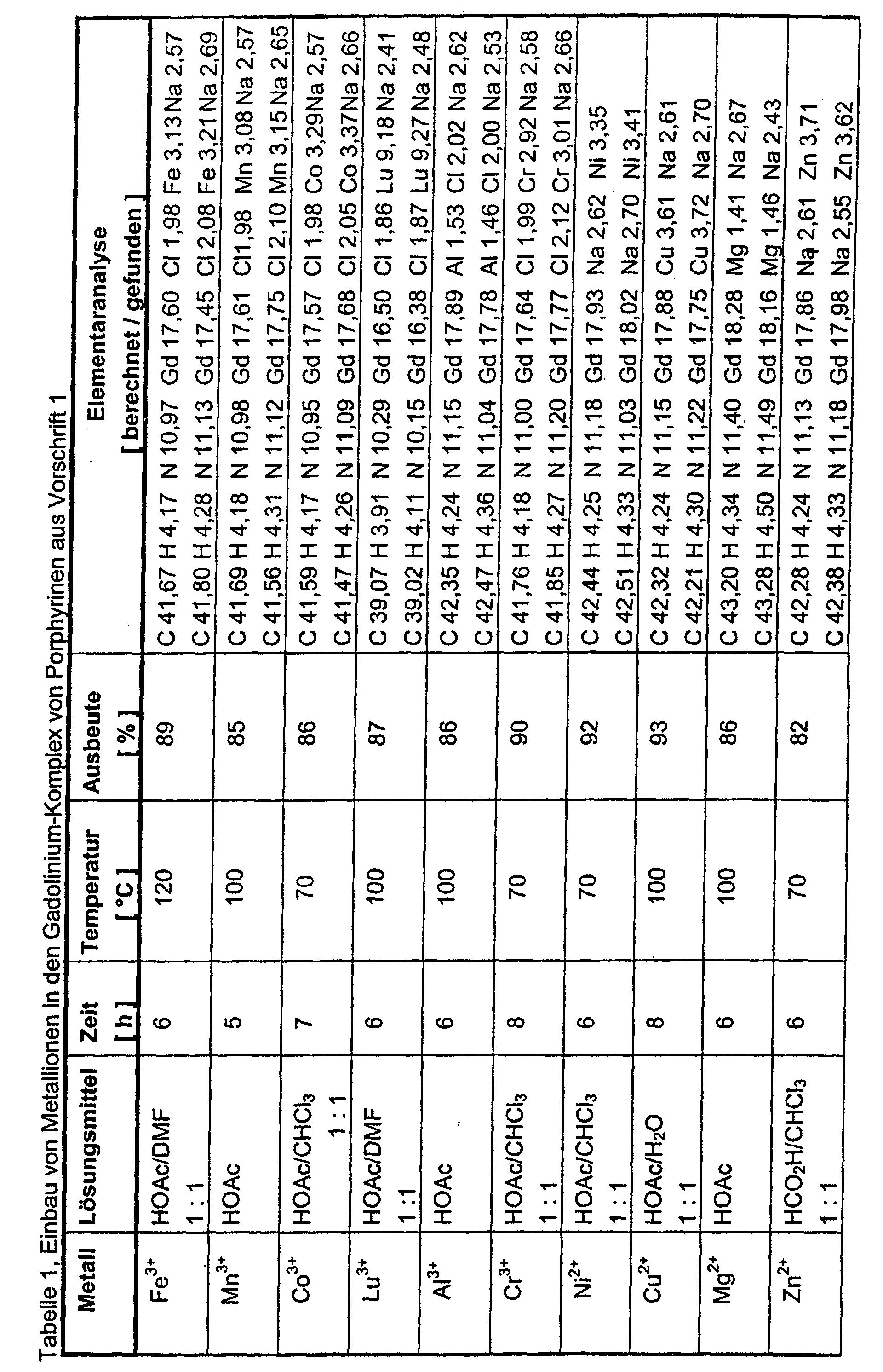

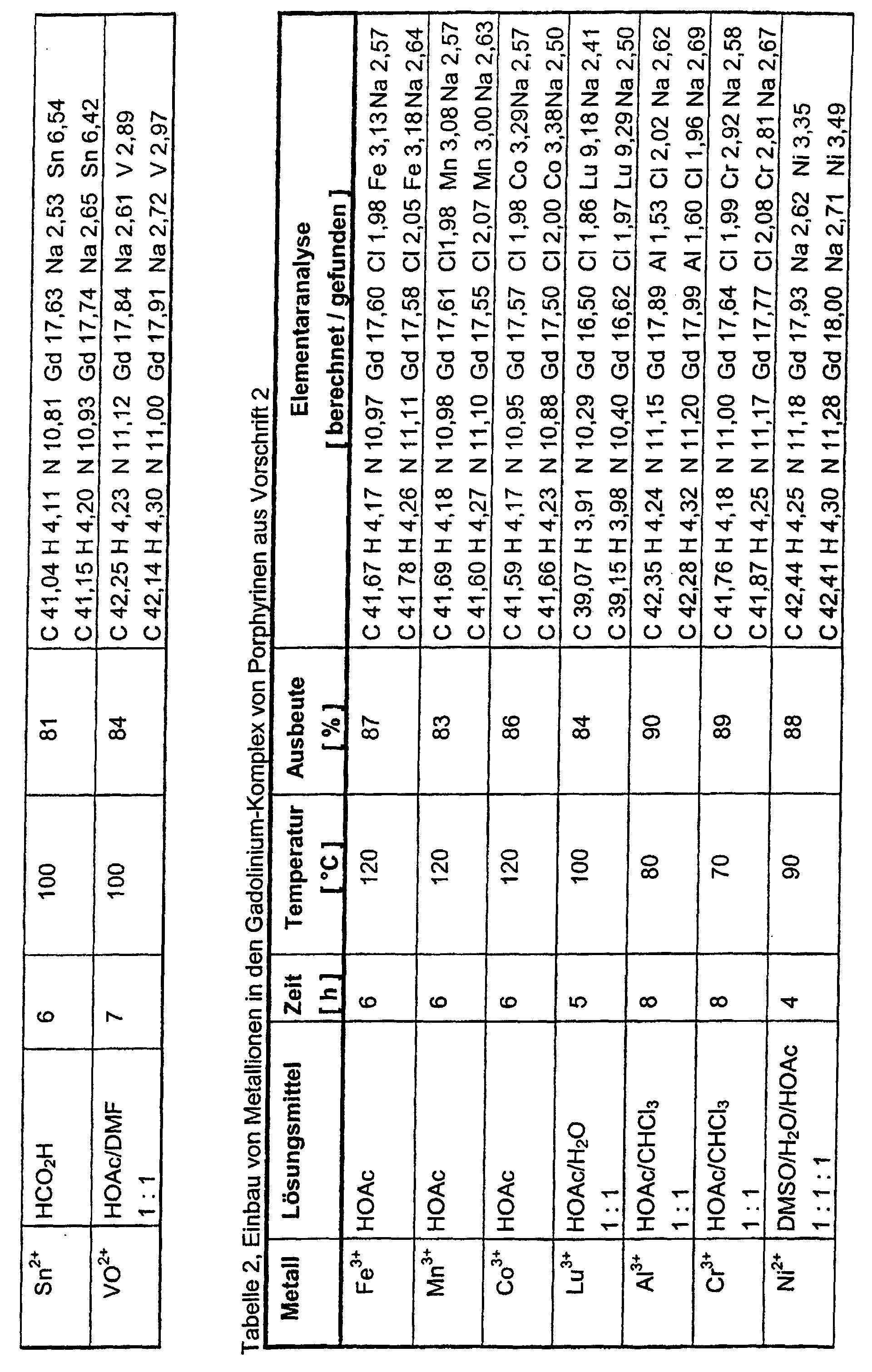

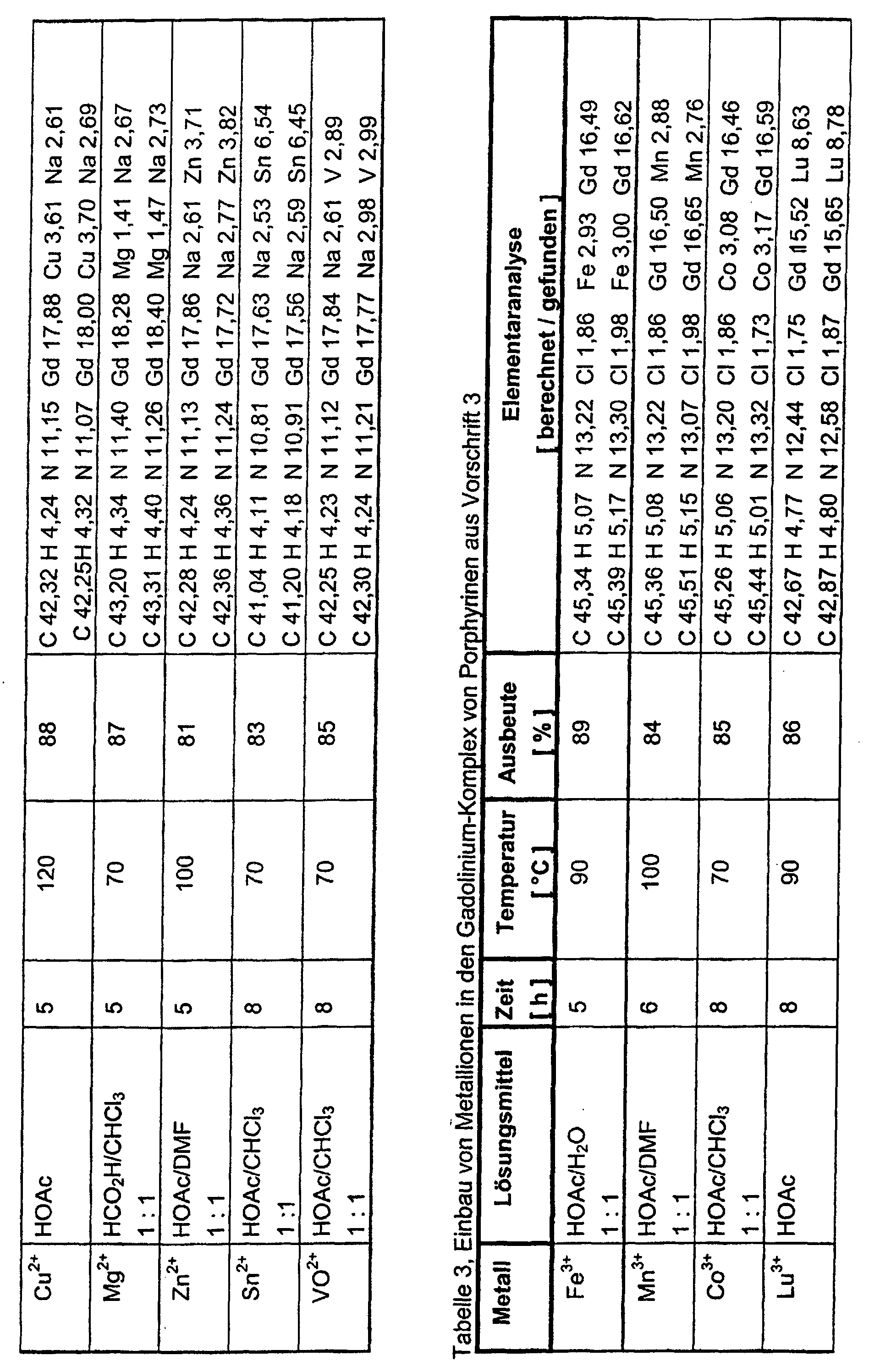

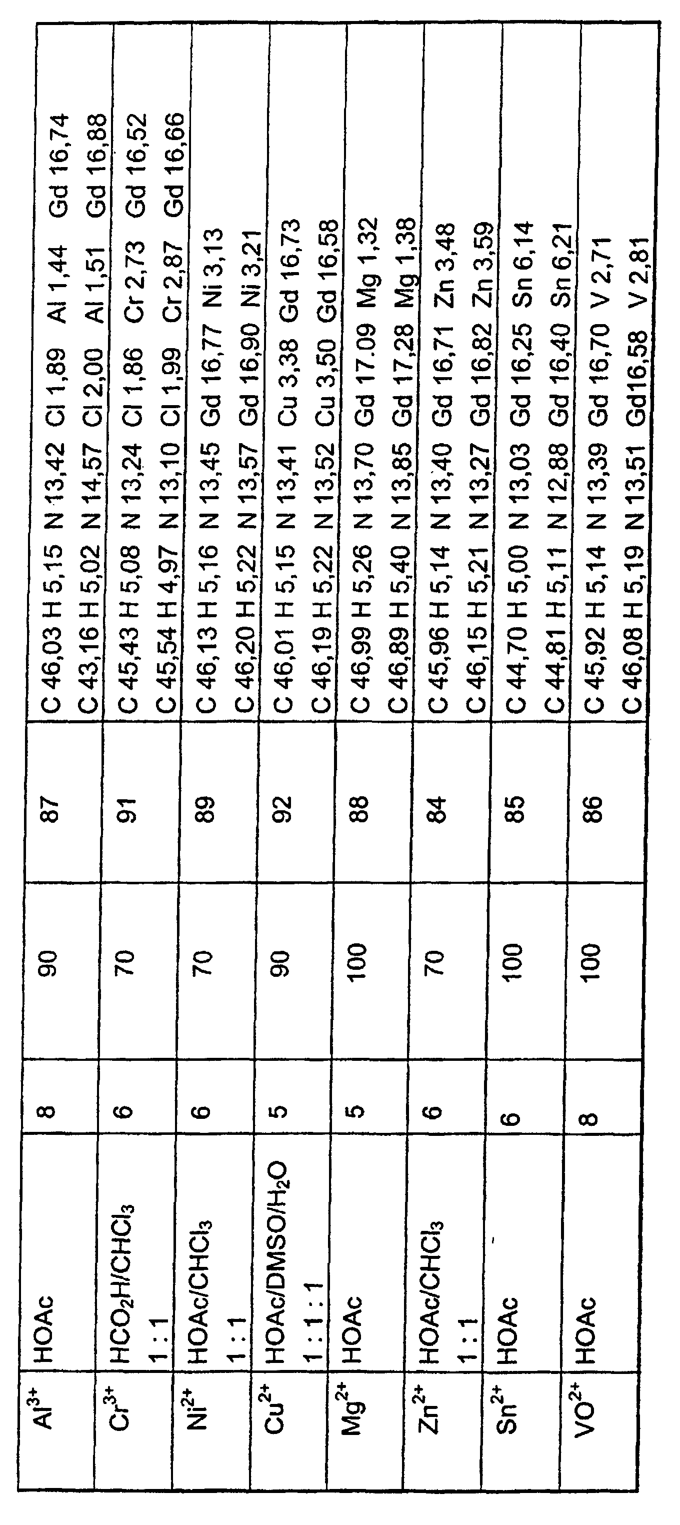

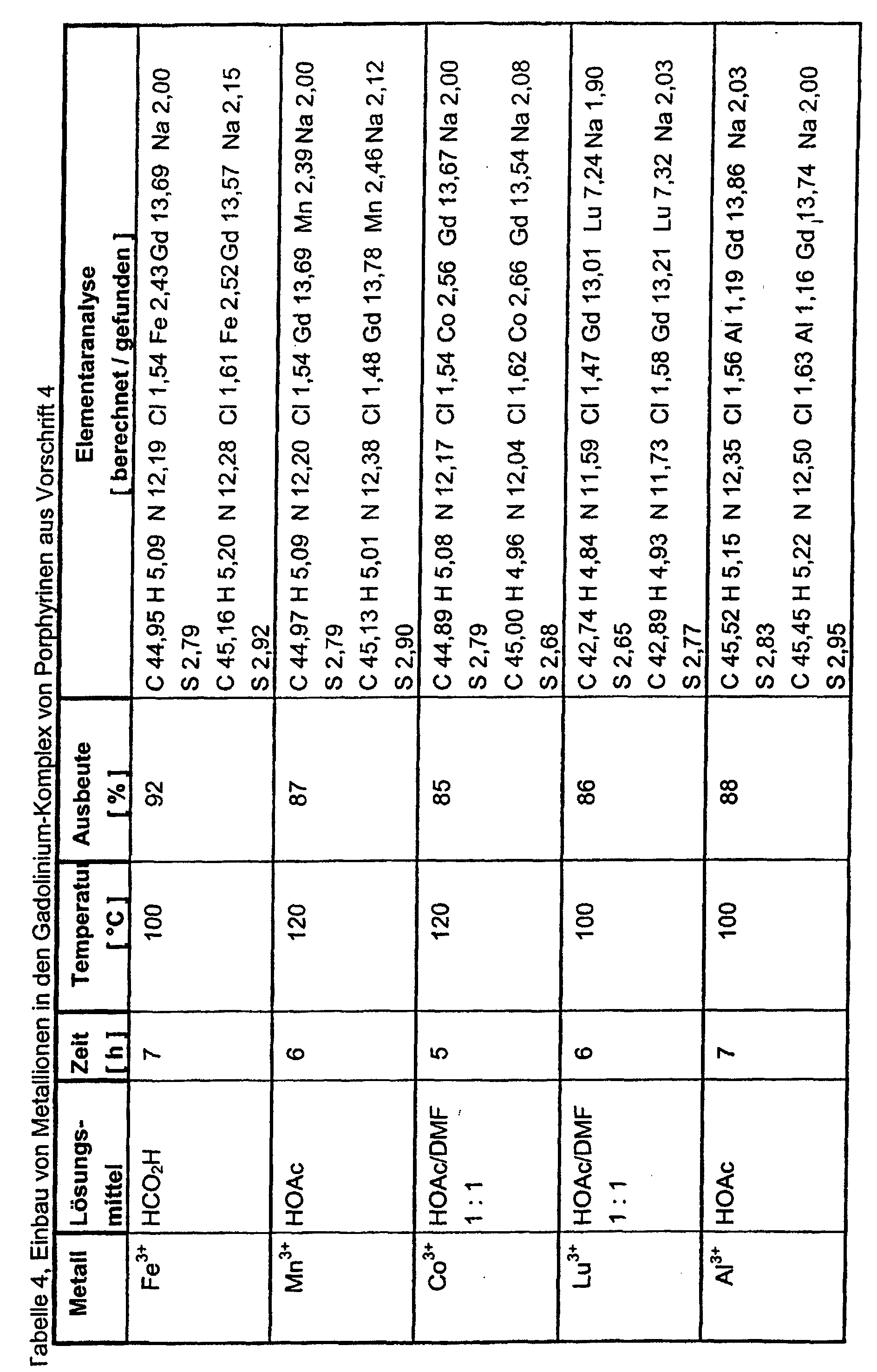

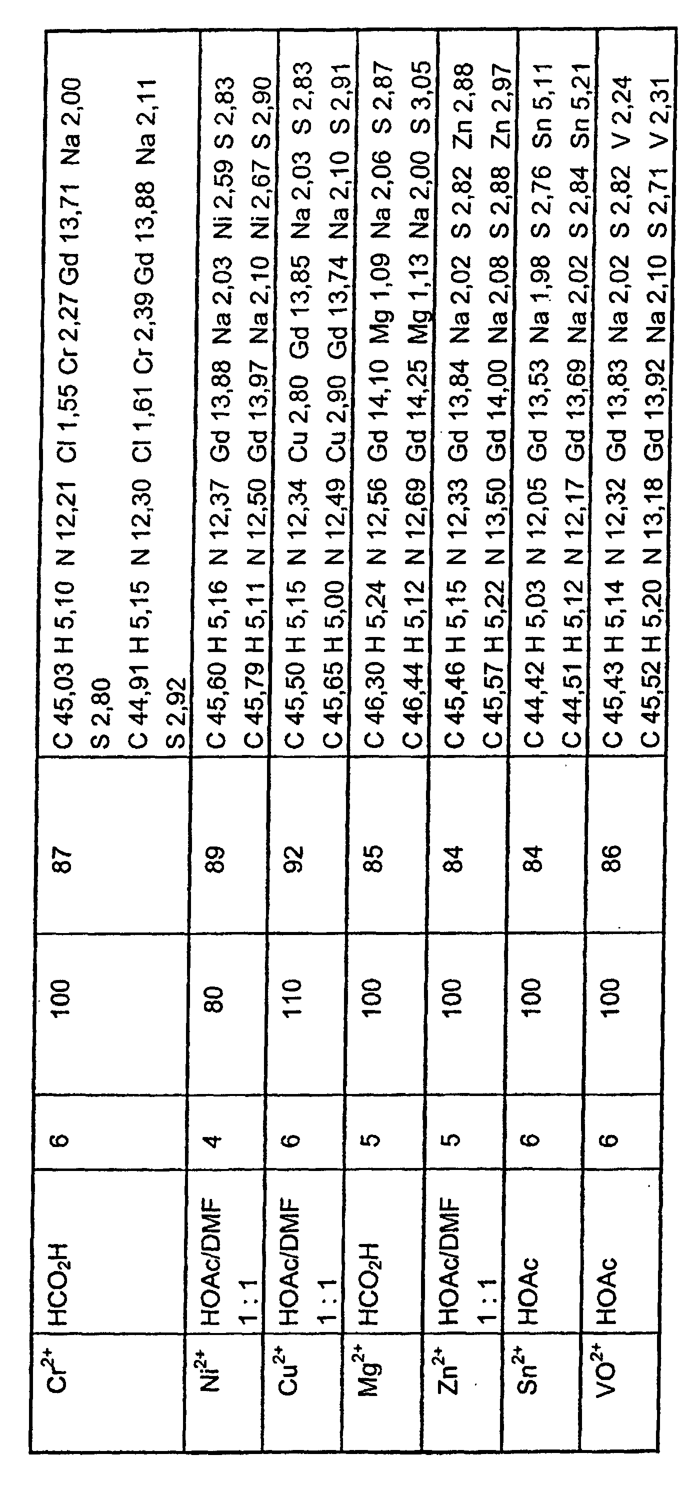

## Patentansprüche

1. Verfahren zur Herstellung von Metalloporphyrin-Metallkomplex-Konjugaten, **dadurch gekennzeichnet, daß** ein Porphyrin-Metallkomplex-Konjugat durch Umsetzung mit einem Metallacetylacetonat in einem protischen oder aprotischen polaren Lösungsmittel oder Lösungsmittelgemisch bei Temperaturen von Raumtemperatur bis 150°C in Konzentrationen von 3-30% in ein Metalloporphyrin-Metallkomplex-Konjugat überführt wird.

2. Verfahren zur Herstellung von Metalloporphyrin-Metallkomplex-Konjugaten der allgemeinen Formel I 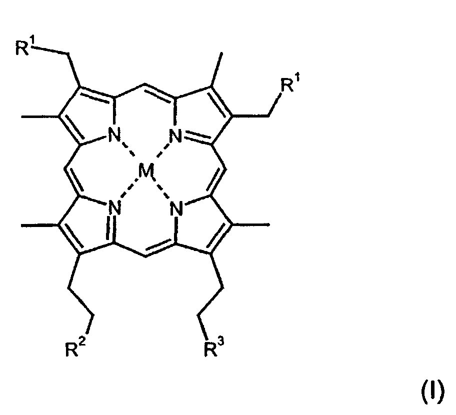 enthaltend mindestens ein Ion eines Elements der Ordnungszahl 20-32, 37-39, 42-51 oder 57-83 im Komplexteil des Moleküls,
worin
M für ein Ion der Metalle Magnesium, Aluminium, Chrom, Mangan, Eisen, Cobalt, Nickel, Kupfer, Zink, Gallium, Technetium, Indium, Zinn, Europium, Lutetium, Tallium, Wismut und für Oxovanadium(IV),
R¹ für ein Wasserstoffatom, für einen geradkettigen C₁-C₆-Alkylrest, einen C₇-C₁₂-Aralkylrest oder für eine Gruppe OR' steht, wobei
R' ein Wasserstoffatom oder ein C₁-C₃-Alkylrest ist,
R² für R³, eine Gruppe -CO-Z oder eine Gruppe -(NH)ₒ-(A)_{q}-NH-D steht,
wobei
Z eine Gruppe -OL ist, mit L in der Bedeutung eines anorganischen oder organischen Kations oder eines C₁-C₄-Alkylrestes,
A eine Phenylenoxy- oder eine durch ein oder mehrere Sauerstoffatome unterbrochene C₁-C₁₂-Alkylen- oder
o und q C₇-C₁₂-Aralkylengruppe bedeutet, unabhängig voneinander die Ziffern 0 oder 1 bedeuten,
D ein Wasserstoffatom oder eine Gruppe -CO-A-(COOL)ₒ-(H)ₘ bedeutet, mit m gleich 0 oder 1 und unter der Maßgabe, daß die Summe aus m und o gleich 1 ist,
R³ für eine Gruppe -(C=Q)(NR⁴)ₒ-(A)_{q}-(NR⁵)-K steht,
wobei
Q für ein Sauerstoffatom oder für zwei Wasserstoffatome steht,
R⁴ eine Gruppe -(A)_{q}-H bedeutet und
K einen Komplexbildner der allgemeinen Formel (IIa), (IIb), (IIc), (IId) oder (IIe) bedeutet, und
R⁵ für den Fall, daß K ein Komplexbildner der Formel (IIa) ist, die gleiche Bedeutung wie R⁴ hat und für den Fall, daß K ein Komplexbildner der Formel (IIb), (llc), (IId) oder (IIe) ist, die gleiche Bedeutung wie D hat,
mit der Maßgabe, daß eine direkte Sauerstoff-Stickstoff Bindung nicht zugelassen ist
und K für einen Komplexbildner der allgemeinen Formeln (IIa), (IIb), (llc), (IId) oder (IIe) 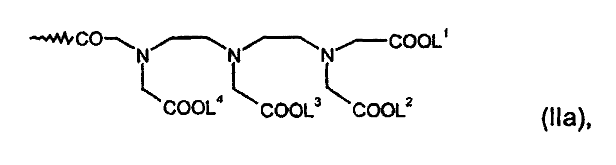 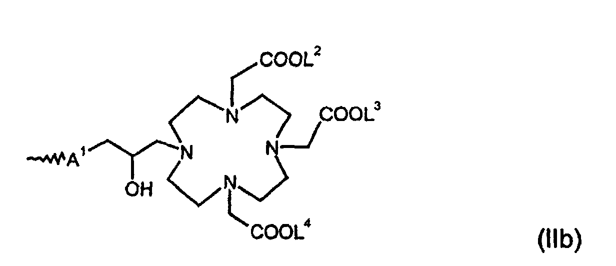 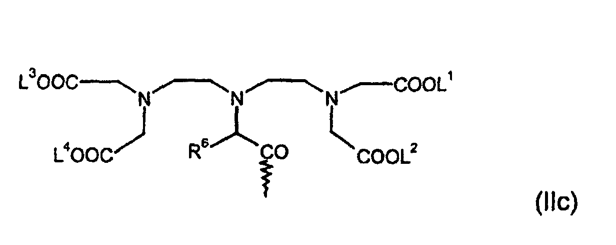 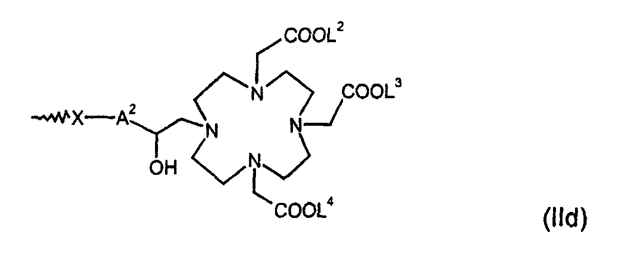 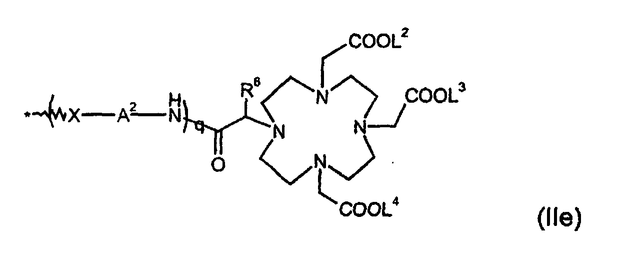 steht,
worin
o und q die oben angegebene Bedeutung haben,
A¹ die für A angegebene Bedeutung hat,
R⁶ für ein Wasserstoffatom, eine geradkettige oder verzweigte C₁-C₇-Alkylgruppe, eine Phenyl- oder Benzylgruppe.
A² für eine Phenylen-,
eine -CH₂-NHCO-CH₂-CH(CH₂COOH)-C₆H₄-β,
eine Phenylenoxy-, oder eine gegebenenfalls durch ein oder mehrere Sauerstoffatome, 1 bis 3 -NHCO- und/oder 1-3-CONH-Gruppen unterbrochene und/oder mit 1 bis 3 -(CH₂)ₒ₋₅COOH-Gruppen substituierte C₁-C₁₂-Alkylen- oder C₇-C₁₂-Aralkylengruppe,
wobei β für die Bindungsstelle an X steht,
X für eine -CO- oder NHCS-Gruppe und
L¹, L², L³ und L⁴ unabhängig voneinander für ein Metallionenäquivalent eines Elements der oben genannten Ordnungszahl steht, unter den Maßgaben, daß mindestens zwei dieser Substituenten für ein Metallionenäquivalent stehen, und daß zum Ausgleich gegebenenfalls vorhandener Ladungen im Metalloporphyrin-Metallkomplex-Konjugat weitere Anionen vorhanden sind und worin freie, nicht zur Komplexbildung benötigte Carbonsäuregruppen auch als Salze mit physiologisch verträglichen anorganischen und/oder organischen Kationen oder als Ester oder als Amide vorliegen können,
**dadurch gekennzeichnet, daß**
Porphyrin-Metallkomplex-Konjugate der allgemeinen Formel (III) 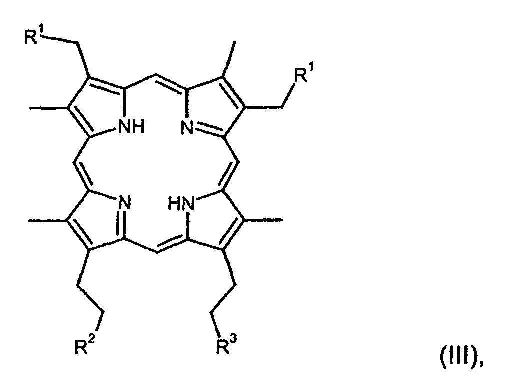 worin R¹, R² und R³ die oben angegebenen Bedeutungen haben,
durch Umsetzung mit einem Metallacetylacetonat des Metalls M, wobei M die oben angegebene Bedeutung hat, in einem protischen oder aprotischen polaren Lösungsmittel oder Lösungsmittelgemisch bei Temperaturen von Raumtemperatur bis 150°C in Konzentrationen von 3-30% in Metalloporphyrin-Metallkomplex-Konjugate der Formel (I) überführt werden.

3. Verfahren nach Anspruch 2, **dadurch gekennzeichnet, daß** das Metall M für Mangan, Eisen, Kupfer oder Zink steht.

4. Verfahren nach Anspruch 2, **dadurch gekennzeichnet, daß** das Lösungsmittel Wasser, Ameisensäure, Essigsäure, Dimethylformamid, Dimethylsulfoxid, Chloroform oder ein Gemisch aus mindestens zwei dieser Lösungsmittel ist.

5. Verfahren nach Anspruch 2, **dadurch gekennzeichnet, daß** Temperaturen von 50°-130°C eingehalten werden.

6. Die Metalloporphyrin-Metallkomplex-Konjugate
{µ-[{16,16'-{Zink(II)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}-digadolinato(2-), Dinatrium,
{µ-[{16,16'-[Acetatomangan(III)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}digadolinato(2-), Dinatrium,
{µ-[{16,16'-[Acetatoeisen(III)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}digadolinato(2-), Dinatrium und
{µ-[{16,16'-[Kupfer(II)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}digadolinato(2-), Dinatrium.

## Claims

1. Process for the production of metalloporphyrin-metal complex conjugates **characterized in that** a porphyrin-metal complex conjugate is converted into a metallporphyrin-metal complex conjugate by reaction with a metal acetylacetonate in a protic or aprotic polar solvent or solvent mixture at temperatures from room temperature to 150°C in concentrations of 3-30%.

2. Process for the production of metalloporphyrin-metal complex conjugates of general formula I 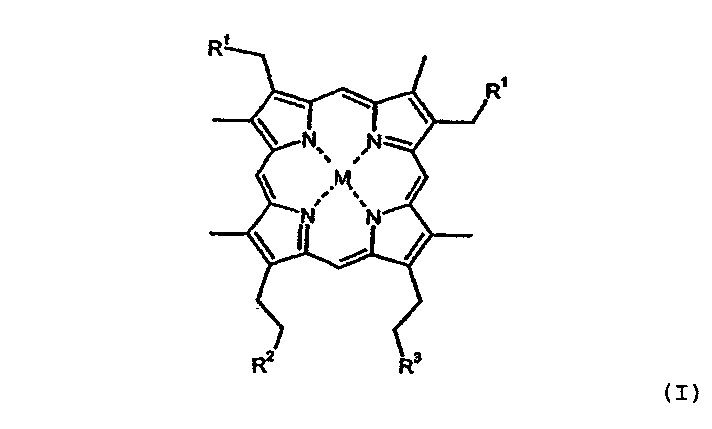 that contain at least one ion of an element of atomic numbers 20-32, 37-39, 42-51 or 57-83 in the complex part of the molecule, in which
M stands for an ion of metals magnesium, aluminium, chromium, manganese, iron, cobalt, nickel, copper, zinc, gallium, technetium, indium, tin, europium, lutetium, thallium, bismuth or oxovanadium(IV),
R¹ stands for a hydrogen atom, a straight-chain C₁-C₆ alkyl radical, a C₇-C₁₂ aralkyl radical or for a group OR',
where R¹ is a hydrogen atom or a C₁-C₃ alkyl radical,
R² stands for R³, a group -CO-Z or a group - (NH)ₒ-(A)_{q}-NH-D.
where Z is a group -OL, with L meaning an inorganic or organic cation or a C₁-C₄ alkyl radical,
A means a phenyleneoxy group or a C₁-C₁₂ alkylene or C₇-C₁₂ aralkylene group that is interrupted by one or more oxygen atoms,
o and q, independently of one another, mean the numbers 0 or 1,
D means a hydrogen atom or a group -CO-A-(COOL)ₒ-(H)ₘ, with m equal to 0 or 1 and provided that the sum of m and o is equal to 1,
R³ stands for a group -(C=Q)(NR⁴)ₒ-(A)_{q}-(NR⁵)-K,
where Q stands for an oxygen atom or for two hydrogen atoms,
R⁴ means an -(A)_{q} -H group, and
K means a complexing agent of general formula (IIa), (IIb), (IIc), (IId) or (IIe), and
R⁵, if K is a complexing agent of formula (IIa), has the same meaning as R⁴, and if K is a complexing agent of formula (IIb), (IIc), (IId) or (IIe), R⁵ has the same meaning as D,
provided that a direct oxygen-nitrogen bond is not allowed
and K stands for a complexing agent of general formula (IIa), (IIb), (IIc), (IId) or (IIe) 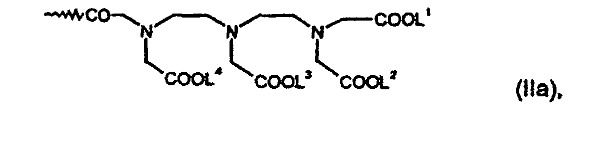 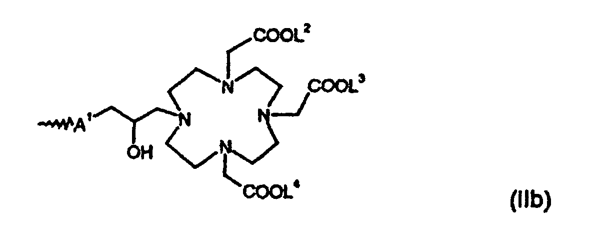 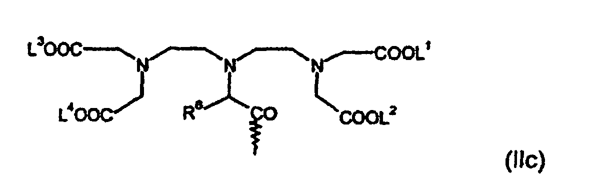 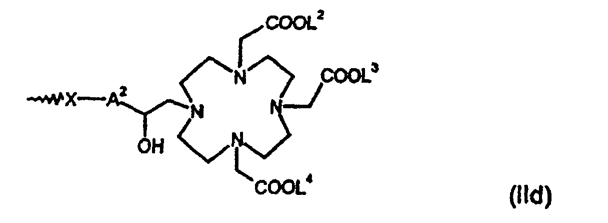 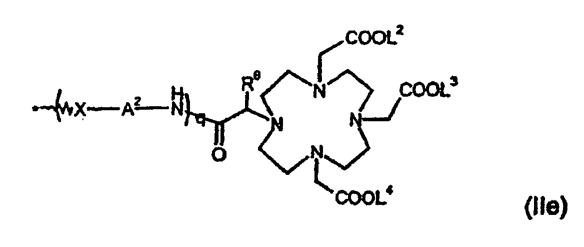 in which
o and q have the above-indicated meanings,
A¹ has the meaning that is indicated for A,
R⁶ stands for a hydrogen atom, a straight-chain or branched C₁-C₇ alkyl group, a phenyl or benzyl group,
A² stands for a phenylene group, a-CH₂-NHCO-CH₂-CH(CH₂COOH)-C₆H₄-β group, a phenyleneoxy group or a C₁-C₁₂ alkylene or C₇-C₁₂ alkylene group that is optionally interrupted by one or more oxygen atoms, 1 to 3 -NHCO and/or 1-3 CONH groups and/or substituted with 1 to 3 -(CH₂)₀₋₅COOH groups,
where β stands for the binding site to X,
X stands for a -CO- or NHCS group, and
L¹, L², L³ and L⁴, independently of one another, stand for a metal ion equivalent of an element of the abovementioned atomic numbers, provided that at least two of these substituents stand for a metal ion equivalent, and other anions are present to compensate for optionally present charges in the metalloporphyrin-metal complex conjugate and in which free carboxylic acid groups that are not required for complexing can also be present as salts with physiologically compatible inorganic and/or organic cations, or as esters or as amides;
**characterized in that**
porphyrin-metal complex conjugates of general formula (III) 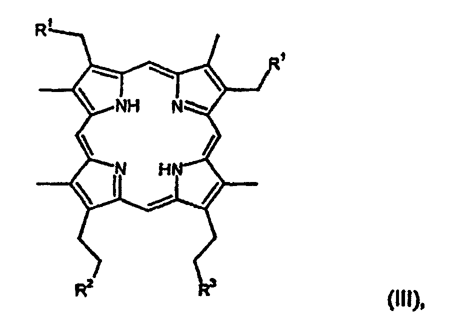 in which R¹, R², R³ and R⁴ have the above-indicated meanings, are converted into metalloporphyrin-metal complex conjugates of formula (I) by reaction with a metal acetylacetonate of metal M, where M has the above-indicated meaning, in a protic or aprotic polar solvent or solvent mixture at temperatures from room temperature to 150°C in concentrations of 3-30%.

3. Process according to Claim 2, **characterized in that** metal M stands for manganese, iron, copper or zinc.

4. Process according to Claim 2, **characterized in that** the solvent is water, formic acid, acetic acid, dimethylformamide, dimethyl sulphoxide, chloroform or a mixture of at least two of these solvents.

5. Process according to Claim 2, **characterized in that** temperatures of 50°-130°C are maintained.

6. The metalloporphyrin-metal complex conjugates
{µ-[{16,16'-[zinc(II)-7,12-diethyl-3,8,13,17-tetramethylporphyrin-2,18-diyl]-bis[3,6,9,-tris(carboxylmethyl)-11,14-dioxo-3,6,9,12,13-pentaazahexadecanoato]}(8-)]}-digadolinato(2-), disodium,
{µ-[{16,16'-[acetatomanganese(III)-7,12-diethyl-3,8,13,17-tetramethylporphyrin-2,18-diyl]-bis-[3,6,9-tris(carboxymethyl)-11,14-dioxo-3,6,9,12,13-pentaazahexadecanoato]}-(8-)]}-digadolinato(2-), disodium,
{µ-[{16,16'-[acetatoiron(III)-7,12-diethyl-3,8,13,17-tetramethylporphyrin-2,18-diyl]-bis[3,6,9-tris(carboxymethyl)-11,14-dioxo-3,6,9,12,13-pentaazahexadecanoato]}(8-)]}-digadolinato(2-), disodium, and
{µ-[{16,16'-[copper(II)-7,12-diethyl-3,8,13,17-tetramethylporphyrin-2,18-diyl]-bis[3,6,9-tris-(carboxymethyl)-11,14-dioxo-3,6,9,12,13-pentaazahexadecanoato]}(8-)]}digadolinato(2-), disodium.

## Revendications

1. Procédé de production de complexes conjugués métalloporphyrine-métal, **caractérisé en ce qu'**un complexe conjugué porphyrine-métal est transformé en un complexe conjugué métalloporphyrine-métal par réaction avec un acétylacétonate de métal dans un solvant ou un mélange de solvants polaires, protiques ou aprotiques, à des températures allant de la température ambiante jusqu'à 150°C en concentrations de 3 à 30 %.

2. Procédé de production de complexes conjugués métalloporphyrine-métal de formule générale I 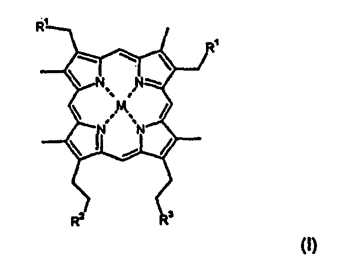 renfermant au moins un ion d'un élément de numéros atomiques 20-32, 37-39, 42-51 ou 57-83 dans la partie complexe de la molécule, dans laquelle
M représente un ion de métaux magnésium, aluminium, chrome, manganèse, fer, cobalt, nickel, cuivre, zinc, gallium, technétium, indium, étain, europium, lutétium, thallium, bismuth ou oxovanadium (IV),
R¹ représente un atome d'hydrogène, un radical alkyle en C₁-C₆ linéaire, un radical aralkyle en C₇-C₁₂ ou un groupe OR',
où R¹ est un atome d'hydrogène ou un radical alkyle en C₁-C₃,
R² représente R³, un groupe -CO-Z ou un groupe -(NH)ₒ-(A)_{q}-NH-D,
où
Z est un groupe -OL, L représentant un cation inorganique ou organique ou un radical alkyle en C₁₋₄,
A représente un groupe phénylène-oxy ou un groupe alkylène en C₁-C₁₂ ou aralkylène en C₇-C₁₂ qui est interrompu par un ou plusieurs atomes d'oxygène,
o et q, indépendamment l'un de l'autre, représentent les nombres 0 ou 1,
D représente un atome d'hydrogène ou un groupe -CO-A-(COOL)ₒ-(H)ₘ, m valant 0 ou 1 et à condition que la somme de m et o soit égale à 1,
R³ représente un groupe -(C=Q)(NR⁴)ₒ-(A)_{q}-(NR⁵)- K,
où
Q représente un atome d'oxygène ou deux atomes d'hydrogène,
R⁴ représente un groupe -(A)_{q}-H, et
K représente un agent complexant de formules générales (IIa), (IIb), (IIc), (IId) ou (IIe), et
R⁵, si K est un agent complexant de formule (IIa), présente la même signification que R⁴, et si K est un agent complexant de formule (IIb), (IIc), (IId) ou (IIe), R⁵ présente la même signification que D,
à condition qu'une liaison directe oxygène-azote ne soit pas permise,
et K représente un agent complexant de formules générales (IIa), (IIb), (IIc), (IId) ou (IIe) 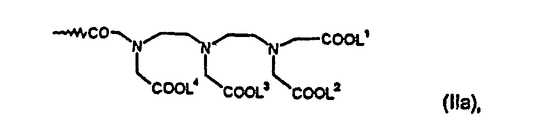 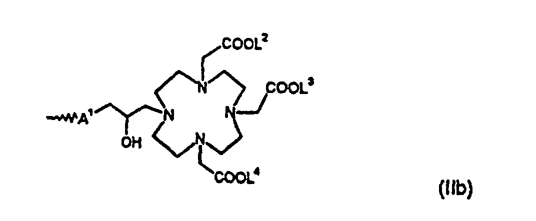 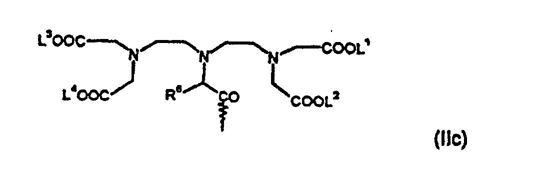 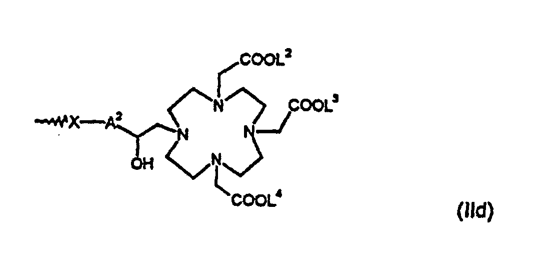 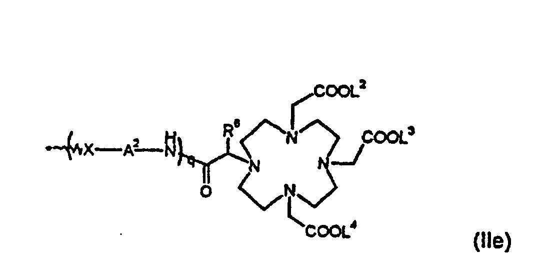 dans lesquelles
o et q présentent la signification indiquée ci-dessus,
A¹ présente la signification indiquée pour A,
R⁶ représente un atome d'hydrogène, un groupe alkyle en C₁-C₇ linéaire ou ramifié, un groupe phényle ou benzyle,
A² représente un groupe phénylène, un groupe -CH₂-NHCO-CH₂-CH (CH₂COOH) -C₆H₄-β, un groupe phénylène-oxy ou un groupe alkylène en C₁-C₁₂ ou aralkylène en C₇-C₁₂ qui est éventuellement interrompu par un ou plusieurs atomes d'oxygène, 1 à 3 groupes NHCO et/ou 1 à 3 groupes CONH et/ou substitué par 1 à 3 groupes - (CH₂)ₒ₋₅COOH,
où β représente le site de liaison à X,
X représente un groupe -CO- ou NHCS, et
L¹, L², L³ et L⁴, indépendamment les uns des autres, représentent un équivalent d'ion métallique d'un élément des numéros atomiques susmentionnés, à condition qu'au moins deux de ces substituants représentent un équivalent d'ion métallique, et que d'autres anions soient présents pour compenser des charges éventuellement présentes dans le complexe conjugué métalloporphyrine-métal, et dans lequel des groupes acide carboxylique libres qui ne sont pas requis pour la complexation peuvent également être présents sous forme de sels avec des cations inorganiques et/ou organiques physiologiquement compatibles ou sous forme d'esters ou d'amides,
**caractérisé en ce que**
des complexes conjugués porphyrine-métal de formule générale (III) 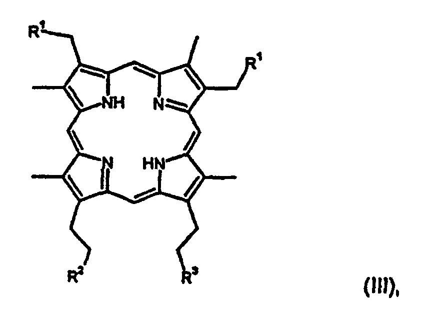 dans laquelle R¹, R² et R³ présentent les significations indiquées ci-dessus, sont transformés en complexes conjugués métalloporphyrine-métal de formule (I) par réaction avec un acétylacétonate de métal M, où M présente la signification indiquée ci-dessus, dans un solvant ou un mélange de solvants polaires, protiques ou aprotiques, à des températures allant de la température ambiante jusqu'à 150°C en concentrations de 3 à 30 %.

3. Procédé selon la revendication 2, dans lequel le métal M représente le manganèse, le fer, le cuivre ou le zinc.

4. Procédé selon la revendication 2, dans lequel le solvant est l'eau, l'acide formique, l'acide acétique, le diméthylformamide, le diméthylsulfoxyde, le chloroforme ou un mélange d'au moins deux de ces solvants.

5. Procédé selon la revendication 2, dans lequel des températures de 50° à 130°C sont maintenues.

6. Complexes conjugués métalloporphyrine-métal
{µ-[{16,16'-{zinc(II)-7,12-diéthyl-3,8,13,17-tétraméthylporphyrine-2,18-diyl]-bis[3,6,9-tris(carboxyméthyl)-11,14-dioxo-3,6,9,12,13-pentaazahexadécanoato]}(8-)]}-digadolinato(2-), disodique,
{µ-[{16,16'-[acétatomanganèse(III)-7,12-diéthyl-3,8,13,17-tétraméthylporphyrine-2,18-diyl]-bis[3,6,9-tris(carboxyméthyl)-11,14-dioxo-3,6,9,12,13-pentaazahexadécanoato]}(8-)]}-digadolinato(2-), disodique,
{µ-[{16,16'-[acétatofer(III)-7,12-diéthyl-3,8,13,17-tétraméthylporphyrine-2,18-diyl]-bis[3,6,9-tris-(carboxyméthyl)-11,14-dioxo-3,6,9,12,13-pentaazahexadécanoato]}(8-)]}-digadolinato(2-), disodique, et
{µ-[{16,16'-[cuivre(II)-7,12-diéthyl-3,8,13,17-tétraméthylporphyrine-2, 18-diyl]-bis[3,6,9-tris(carboxyméthyl)-11,14-dioxo-3,6,9,12,13-pentaazahexadécanoato]}(8-)]}-digadolinato(2-), disodique.
